(19) Europäisches Patentamt
European Patent Office
Office européen des brevets

(11) EP 0 802 181 B1

(12) EUROPÄISCHE PATENTSCHRIFT

(45) Veröffentlichungstag und Bekanntmachung des Hinweises auf die Patenterteilung: **22.12.1999 Patentblatt 1999/51**

(51) Int. Cl.[6]: **C07C 403/20**, A61K 31/20, A61K 31/23

(21) Anmeldenummer: **97105715.3**

(22) Anmeldetag: **07.04.1997**

# (54) Neue Retinoide

Retinoids

Rétinoides

(84) Benannte Vertragsstaaten:
**AT BE CH DE DK ES FI FR GB GR IE IT LI LU MC NL PT SE**

(30) Priorität: **15.04.1996 EP 96105849**

(43) Veröffentlichungstag der Anmeldung: **22.10.1997 Patentblatt 1997/43**

(73) Patentinhaber:
**F. HOFFMANN-LA ROCHE AG**
**4070 Basel (CH)**

(72) Erfinder:
- **Klaus, Michael**
  **79576 Weil am Rhein (DE)**
- **Mohr, Peter**
  **4054 Basel (CH)**

(74) Vertreter:
**Kjellsaa-Berger, Hanny, Dr. et al**
**Grenzacherstrasse 124,**
**Postfach 3255**
**4070 Basel (CH)**

(56) Entgegenhaltungen:
**EP-A- 0 010 208** **GB-A- 2 156 676**

- **HELV. CHIM. ACTA (HCACAV,0018019X);80; VOL.63 (6); PP.1467-72, F. HOFFMANN-LA ROCHE UND CO., A.-G.;ABT. VITAM. ERNAEHRUNGSFORSCH.; BASEL; CH-4002; SWITZ., XP002034934 MAYER H ET AL: "Synthesis of optically active natural carotenoids and structurally related natural products. VII. Synthesis of (3R)-3-hydroxyretinol, (3R)-3-hydroxyretinal and (3R)-3-hydroxyretinoic acid"**
- **CHEM. PHARM. BULL. (CPBTAL,00092363);88; VOL.36 (1); PP.78-86, KOBE WOMEN'S COLL. PHARM.;KOBE; 658; JAPAN (JP), XP000676609 ITO M ET AL: "Retinoids and related compounds. X. Synthesis of geometrical isomers of (.+-.)-2-and (.+-.)-3-hydroxyretinals and identification of the chromophore of the fly visual pigment"**
- **PHARM. RES. (PHREEB,07248741);90; VOL.7 (3); PP.270-3, OHIO STATE UNIV.;COLL. PHARM.; COLUMBUS; 43210; OH; USA (US), XP002034935 HARTMAN D A ET AL: "Microbial biotransformation of retinoic acid by Cunninghamella echinulata and Cunninghamella blakesleeana"**
- **BIOCHEM. J. (BIJOAK,00062936);77; VOL.168 (3); PP.557-64, UNIV. GAUHATI;DEP. CHEM.; GAUHATI; INDIA, XP002034936 BARUA A B ET AL: "Occurrence of 3-hydroxyretinol in the freshwater fish Bagarius bagarius and Wallago attu. Isolation and synthesis"**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7/3.6

## Beschreibung

Die vorliegende Erfindung betrifft neue Retinoide der allgemeinen Formel

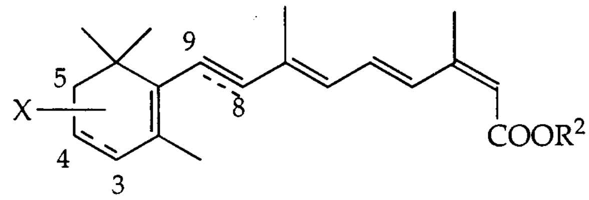

I

worin

X Oxo oder ein Rest $OR^1$ in 4- oder 5-Stellung;
$R^1$ Wasserstoff, Alkyl oder Acyl; und
$R^2$ Wasserstoff oder Alkyl ist;

die punktierten Bindungen fakultativ sind und eine 3,4-Doppelbindung nur anwesend sein kann, wenn X in 5-Stellung ist;
sowie pharmazeutisch anwendbare Salze von Carbonsäuren der Formel I.

Die Erfindung betrifft weiterhin ein Verfahren zur Herstellung dieser Retinoide und ihrer Salze, diese Derivate oder ihre Salze enthaltende pharmazeutische Präparate, sowie die Verwendung dieser Retinoide und ihrer Salze als Heilmittel oder zur Herstellung von Heilmitteln.

**Pharmazeutische Präparate enthaltend Vitamin A Derivate sind bekannt, so sind z.B. in GB A 2 156 676 Präparate enthaltend 4-Oxo- und 4-Hydroxy-13-cis-Vitamin A Derivate zur Behandlung von Akne und Seborrhoea beschrieben. Ein Verfahren zur Herstellung von optisch aktiven Vitamin A Derivaten ist in Helv. Chim. Acta 1980, 63(6), 1467 beschrieben.**

In den Verbindungen der allgemeinen Formel I sind Alkylgruppen vorzugsweise niedere Alkylgruppen (wobei "nieder" Gruppen mit 1-7 C-Atomen bezeichnet), die geradkettig oder verzweigt sein können, wie Methyl, Aethyl, Propyl, Isopropyl, Butyl, Isobutyl, tert.-Butyl, Pentyl, Hexyl und Heptyl. Der Ausdruck Acyl bezieht sich auf vorzugsweise niedere Alkanoylreste wie Acetyl, Propionyl, Butyryl und Pivaloyl; aromatische Acylreste wie Benzoyl und Toluyl; und araliphatische Acylreste wie Phenylacetyl.

Verbindungen der Formel I, in denen X ein Rest OR1 ist, können als R- oder S-Enantiomere oder als Racemate vorliegen.

Eine bevorzugte Gruppe von Verbindungen der Formel I sind Verbindungen der Formel

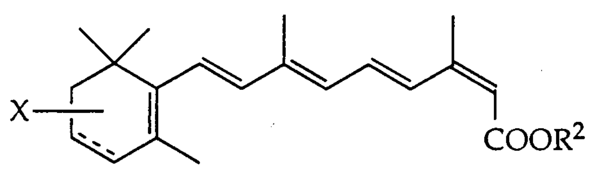

IA

insbesondere solche der Formeln

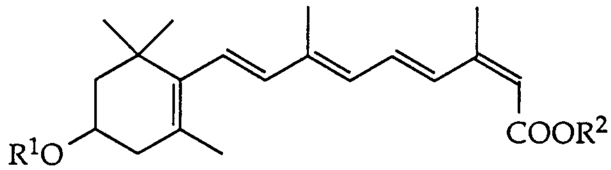

IB

und

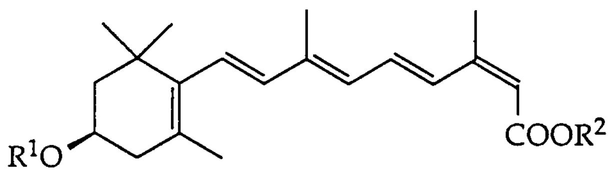

IC

in denen X, $R^1$ und $R^2$ die oben angegebene Bedeutung haben.

Weitere Untergruppen von Verbindungen der Formel I sind die Verbindungen der Formeln ID, IE, IF und IG

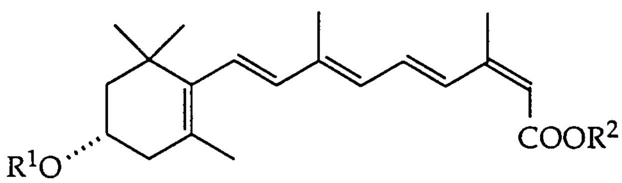

ID

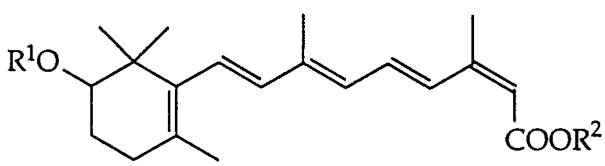

IE

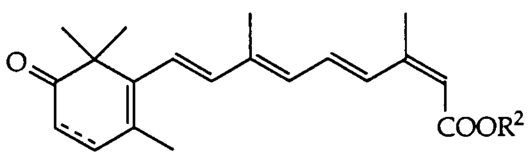

IF

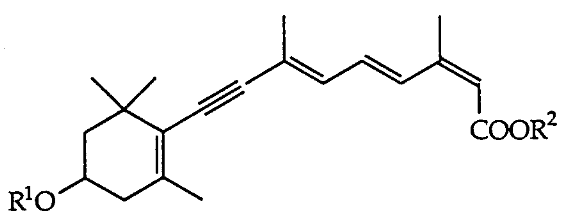

IG

Beispiele von Verbindungen der Formel I sind:

(2Z,4E,6E,8E)-(R)-9-(4-Hydroxy-2,6,6-trimethyl-cyclohex-1-enyl)-3,7-dimethyl-nona-2,4,6,8-tetraensäure,
(2Z,4E,6E)-(R)-9-(4-Hydroxy-2,6,6-trimethyl-cyclohex-1-enyl)-3,7-dimethyl-nona-2,4,6-trien-8-ynsäure,
(2Z,4E,6E,8E)-(R)-9-(4-Hydroxy-2,6,6-trimethyl-cyclohex-1-enyl)-3,7-dimethyl-nona-2,4,6,8-tetraensäure-ethylester,
(2Z,4E,6E,8E)-9-(5-Oxo-2,6,6-trimethyl-cyclohexa-1,3-dienyl)-3,7-dimethyl-nona-2,4,6,8-tetraensäure,

(2Z,4E,6E,8E)-(R)-9-(4-Acetoxy-2,6,6-trimethyl-cyclohex-1-enyl)-3,7-dimethyl-nona-2,4,6,8-tetraensäure,
(2Z,4E,6E,8E)-(R)-9-(4-Methoxy-2,6,6-trimethyl-cyclohex-1-enyl)-3,7-dimethyl-nona-2,4,6,8-tetraensäure,
(2Z,4E,6E,8E)-(RS)-9-(5-Hydroxy-2,6,6-trimethyl-cyclohex-1-enyl)-3,7-dimethyl-nona-2,4,6,8-tetraensäure,
(2Z,4E,6E,8E)-(RS)-9-(5-Hydroxy-2,6,6-trimethyl-cyclohexa-1,3-dienyl)-3,7-dimethyl-nona-2,4,6,8-tetraensäure,
(2Z,4E,6E,8E)-9-(5-Oxo-2,6,6-trimethyl-cyclohex-1-enyl)-3,7-dimethyl-nona-2,4,6,8-tetraensäure,
(2Z,4E,6E,8E)-9-(4-Oxo-2,6,6-trimethyl-cyclohex-1-enyl)-3,7-dimethyl-nona-2,4,6,8-tetraensäure,
(2Z,4E,6E,8E)-(S)-9-(4-Hydroxy-2,6,6-trimethyl-cyclohex-1-enyl)-3,7-dimethyl-nona-2,4,6,8-tetraensäure und
(2Z,4E,6E,8E)-(RS)-9-(4-Hydroxy-2,6,6-trimethyl-cyclohex-1-enyl)-3,7-dimethyl-nona-2,4,6,8-tetraensäure.

Die Verbindungen der Formel I und ihre Salze können erfindungsgemäss dadurch hergestellt werden, dass man eine Verbindung der allgemeinen Formel

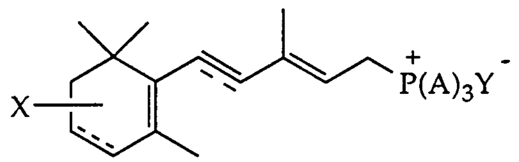

*II*

worin A Phenyl oder substituiertes Phenyl und $Y^-$ ein Anion ist,
und X und die punktierten Bindungen die oben angegebene Bedeutung haben,

mit 5-Hydroxy-4-methyl-5H-furan-2-on in Gegenwart einer Base umsetzt, im Reaktionsprodukt enthaltene Isomere, in denen die bei der Reaktion gebildete C=C-Doppelbindung cis-Konfiguration aufweist, zum trans-Isomeren isomerisiert und gewünschtenfalls das Reaktionsprodukt der Formel I einer oder mehreren der nachfolgenden Operationen unterwirft:

a) Veresterung einer Carboxyl- oder Hydroxygruppe;

b) Verätherung einer Hydroxygruppe;

c) Oxidation einer Hydroxygruppe zur Oxogruppe;

d) Reduktion einer Oxogruppe zur Hydroxygruppe;

e) sterische Invertierung einer Hydroxygruppe; und

f) Ueberführung einer Carboxygruppe in ein Salz.

Die vorstehenden Umsetzungen können nach an sich bekannten Methoden vorgenommen werden.

Die Umsetzung der Verbindung II kann nach den bekannten Methoden der Wittig- Reaktion durchgeführt werden. Dabei werden die Reaktionspartner in Gegenwart eines säurebindenden Mittels, z.B. in Gegenwart einer starken Base, wie KOH in einem wässrigem Lösungsmittel; oder Natriumhydrid, K-tert.Butylat oder Natriumäthylat in einem wasserfreien Lösungsmittel, wie Dimethylformamid oder Methylenchlorid, in einem zwischen etwa -30°C und Raumtemperatur liegenden Temperaturbereich miteinander umgesetzt. Vorzugsweise geht man von Verbindungen der Formel II aus, in denen X Oxo oder Hydroxy ist. Die Wittig-Reaktion liefert ein Gemisch von cis/trans Isomeren der bei der Reaktion gebildeten C=C Doppelbindung. Dieses Gemisch von 4E/Z - Isomeren kann in an sich bekannter Weise, z.B. durch Behandlung mit Pd-Katalysatoren, wie Pd-(II)-nitrat selektiv zum 4E-Isomeren isomerisiert werden.

Von den anorganischen Säureanionen $Y^-$ ist das Chlor- und Brom-ion oder das Hydrosulfat-ion, von den organischen Säureanionen ist das Tosyloxy-ion bevorzugt. Der Rest A ist vorzugsweise Phenyl.

Die Veresterung einer Carboxylgruppe in einer Verbindung der Formel I kann z.B. durch Umsetzung mit einem Alkylhalogenid in Gegenwart einer Base, wie Kaliumcarbonat, in einem organischen Lösungsmittel, wie z.B. Aethylacetat, vorgenommen werden. Die Veresterung einer Hydroxygruppe kann durch Umsetzung mit einem Acylhalogenid in Gegenwart einer Base, wie Pyridin, vorgenommen werden. Eine Hydroxygruppe kann z.B. nach den Methoden von Swern (Dimethylsulfoxid/Oxalylchlorid) oder Dess-Martin (Periodinan) zur Oxogruppe oxidiert werden. Die Reduktion einer Oxogruppe zur Hydroxygruppe kann mit Hydrid-Reduktionsmitteln wie $NaBH_4$ durchgeführt werden. Die sterische Konfiguration einer Hydroxygruppe kann nach der Mitsunobu-Methode (Reaktion mit Triphenylphosphin, p-Nitrobenzoesäure und Azodicarbonsäurediethylester und anschliessende Verseifung invertiert werden.

Beispiele von Salzen, in die die Carbonsäuren der Formel I übergeführt werden können, sind Alkalimetallsalze,

wie Na- und K-Salze, Erdalkalimetallsalze wie Ca- und Mg-Salze, und Animoniumsalze, z.B. Salze mit Alkylaminen und Hydroxyalkylaminen oder mit anderen organischen Basen, wie Dimethylamin, Diäthanolamin und Piperidin.

Die Verbindungen der Formel II können wie in Pure and Appl.Chem. 57, 741 (1985) oder den dort angegebenen weiteren Literaturstellen beschrieben hergestellt werden.

Die erfindungsgemässen Verbindungen können zur Therapie und Prophylaxe von dermatologischen Erkrankungen, die mit Epithelläsionen einhergehen, z.B. Akne und Psoriasis, sowie malignen und prämalignen Epithelläsionen, Tumoren und präcancerösen Veränderungen der Schleimhäute in Mund, Zunge, Kehlkopf, Oesophagus, Blase, Cervix und Colon verwendet werden.

Die Verbindungen der Formel I und deren Salze können dementsprechend in Form pharmazeutischer Präparate Anwendung finden.

Die zur systemischen Anwendung dienenden Präparate können z.B. dadurch hergestellt werden, dass man eine Verbindung der Formel I oder ein Salz davon als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Für die enterale und parenterale Verabreichung können die Verbindungen der Formel I in Mengen von etwa 10-400 mg, vorzugsweise 20-200 mg/Tag, an Erwachsene verabreicht werden.

Zur topischen Anwendung werden die Wirkstoffe zweckmässig in Form von Salben, Tinkturen, Crèmen, Lösungen, Lotionen, Sprays, Suspensionen und dergleichen verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise 0,3-2%ige, Salben oder Crèmen geeignet.

Den Präparaten kann gegebenenfalls ein Antioxydationsmittel, z.B. Tocopherol, N-Methyl-g-tocopheramin sowie butyliertes Hydroxyanisol oder butyliertes Hydroxytoluol beigemischt werden.

Die Wirksamkeit der erfindungsgemässen Verbindungen bei der Behandlung der Akne kann mit den nachstehend beschriebenen Versuchsanordnungen gezeigt werden:

1. Hemmung der Proliferation menschlicher Sebocyten (in vitro) Literatur: Methods in Enzymology 190, 334 (1990), T. Doran and S. Shapiro

2. Aenderung der Differenzierung von Schweinesebocyten (invitro), Induktion von Keratin 7.
   Kulturen von Schweinesebocyten werden aus isolierten Sebumdrüsen erwachsener Minischweine hergestellt. Sie reagieren auf die Behandlung mit Retinoiden mit der Expression von Keratin 7, was auch der in vivo Situation entspricht. Keratin 7 wird als ein Marker für eine modifizierte Differenzierung der Sebocyten angesehen. Der entstehende Phenotyp kann kein Sebum mehr produzieren. Die Messung der Keratin 7-Induktion basiert auf einem ELISA assay unter Verwendung eines Maus-monoklonalen Antikeratin 7-Antikörpers.

3. Minipig-Modell (in vivo)
   Die Haut der Minischweine hat eine ähnliche anatomische Struktur wie menschliche Haut. Im besonderen ähneln die Sebumdrüsen denen menschlicher Aknepatienten. Unter Retinoid-Behandlung zeigen sie die gleichen histologischen Veränderungen wie beim Menschen. Die Schweine erhalten täglich eine orale Dosis der Versuchssubstanz während 8 Wochen. Alle 2 Wochen wird eine Biopsie genommen und histologisch untersucht.

Die im Beispiel 1 hergestellte Verbindung zeigt in diesem Versuch bei einer Dosis von 10 mg/kg bereits nach 3-4 Wochen eine deutliche Verkleinerung der Sebumdrüsen, die nach 8 Wochen weitgehend verschwunden sind. Im Gegensatz zu Isotretinoin treten selbst bei einer Dosis von 50 mg/kg keinerlei Nebenwirkungen auf Haut und Schleimhäute auf.

Teratogene Effekte gehören zu den schwerschwiegensten Nebenwirkungen aller Retinoide. Die hier beanspruchten Verbindungen sind deutlich weniger teratogen als z.B. Isotretinoin oder Tretinoin. Zur Evaluierung des teratogenen Potentials wurde der limb bud cell culture assay verwendet (Literatur: Arch. Toxiol. 60, 403 [1987] A. Kistler). Die Korrelation zwischen $IC_{50}$ in diesem Test und der in vivo Teratogenität ist sehr gut.

| Verbindung von Beispiel | $IC_{50}$ (nM) |
|---|---|
| 1 | >1000 |
| 3 | >1000 |
| 4 | >1000 |
| Isotretinoin | 200 |
| Tretinoin | 80 |

Die Erfindung wird durch die nachstehenden Beispiele weiter erläutert.

Beispiel 1

50 g [(2E,4E)-(R)-5-(4-Hydroxy-2,6,6-trimethyl-1-cyclohexen-1-yl)-3-methyl-2,4-pentadienyl]-triphenylphosphoniumchlorid wurden in 500 ml Isopropanol gelöst und mit 11 g 5-Hydroxy-4-methyl-5H-furan-2-on versetzt. Nach Abkühlen des Reaktionsgemisches auf -30°C wurden 120 ml einer wässrigen 2N KOH-Lösung hinzugetropft und eine weitere Stunde bei -30°C unter Argon gerührt. Das Reaktionsgemisch wurde anschliessend auf 1.3 l Eiswasser gegossen, 6mal mit je 500 ml eines Hexan/Essigester-Gemisches (2:1) extrahiert, die wässrige, alkalische Lösung unter Eiskühlung durch Zugabe von eiskalter 3N Schwefelsäure angesäuert und anschliessend 3mal mit Essigester extrahiert. Die organische Phase wurde mit gesättigter Kochsalz-Lösung gewaschen, über Natriumsulfat getrocknet und eingedampft. Der viskose, gelbe Rückstand wurde erneut in 800 ml Essigester gelöst, mit 100 g Kieselgel (Merck 0,063-0,2 mm) versetzt, während 5 Minuten unter kräftigem Rühren auf dem Dampfbad leicht erwärmt und nach Filtration am Rotationsverdampfer eingeengt. Das so erhaltene gelbe, kristalline Rohprodukt wurde mit 500 ml Acetonitril versetzt und unter Argon auf 50°C erwärmt. Durch portionenweises Zugeben von ca. 700 ml Acetonitril erhielt man eine Klare, gelbe Lösung. Nach Zugabe von 1,1 g Triphenylphosphin und 118 ml einer 0,125%igen Lösung von Palladium(II)-nitrat in Acetonitril wurde das Reaktionsgemisch 3 Stunden bei 50°C gerührt. Beim Abkühlen der Reaktionslösung auf -10°C kristallisierte das Endprodukt aus. Es wurde abfiltriert, mit Hexan gewaschen und am Hochvakuum bei 40°C getrocknet. Umkristallisation aus Hexan/Essigester ergab 15 g (2Z,4E,6E,8E)-(R)-9-(4-Hydroxy-2,6,6-trimethyl-cyclohex-1-enyl)-3,7-dimethyl-nona-2,4,6,8-tetraensäure, Smp. 180-182°C (Zers), $[\alpha]_D^{20}$ = -49,7 (c=1, Dioxan).

Beispiel 2

3 g der nach Beispiel 1 erhaltenen Carbonsäure wurden nacheinander mit 120 ml Methylethylketon, 5,2 g feingemahlenem Kaliumcarbonat und 7,4 g Ethyljodid versetzt und unter Argon und kräftigem Rühren während 2,5 Stunden am Rückfluss erwärmt. Das Reaktionsgemisch wurde auf Eis/1N Salzsäure gegossen, mit Ether extrahiert, mit Wasser gewaschen, getrocknet und eingedampft. Das ölige Rohprodukt wurde über Kieselgel (Eluierungsmittel Hexan/Essigester 2:1) filtriert und aus Hexan umkristallisiert. Man erhielt 2,9 g (2Z,4E,6E,8E)-(R)-9-(4-Hydroxy-2,6,6-trimethylcyclohex-1-enyl)-3,7-dimethyl-nona-2,4,6,8-tetraensäure-ethylester in gelben Kristallen, Smp. 93-95°C, $[\alpha]_D^{20}$ = -45,5 (c=1, Dioxan).

Beispiel 3

In Analogie zu Beispiel 1 wurden durch Umsetzung von 10 g [(E)-(R)-5-(4-Hydroxy-2,6,6-trimethyl-1-cyclohexen-1-yl)-3-methyl-2-penten-4-ynyl]-triphenylphosphoniumchlorid mit 2,3 g 5-Hydroxy-4-methyl-5H-furan-2-on nach Palladium katalysierter Isomerisierung des Rohproduktes und Umkristallisation aus Hexan/Essigester 3,1 g (2Z,4E,6E)-(R)-9-(4-Hydroxy-2,6,6-trimethyl-cyclohex-1-enyl)-3,7-dimethyl-nona-2,4,6-trien-8-yn-säure als gelbe Kristalle erhalten, Smp. 189-190°C (Zers.), $[\alpha]_D^{20}$ = -87,3 (c=1, $CHCl_3$).

Beispiel 4

9 g [(2E,4E)-3-Methyl-5-(2,6,6-trimethyl-5-oxo-1,3-cyclohexadien-1-yl)-2,4-pentadienyl]triphenylphosphoniumbromid und 1,7 g 5-Hydroxy-4-methyl-5H-furan-2-on wurden in 100 ml Isopropanol suspendiert. Nach dem Abkühlen des Reaktionsgemisches auf -30°C wurden 20 ml einer wässrigen 2N Kaliumhydroxid-Lösung hinzugetropft. Nach 30-minütigem Rühren bei -30°C wurde das Reaktionsgemisch auf Eiswasser gegossen, 3mal mit Hexan extrahiert, die

alkalische wässrige Phase mit eiskalter 3N Schwefelsäure angesäuert und mehrfach mit Essigester extrahiert. Die organische Phase wurde mit Wasser gewaschen, getrocknet und eingedampft. Das bräunliche, viskose Oel wurde durch Chromatographie an Kieselgel (Eluierungsmittel Hexan/Essigester 3:1) gereinigt. Das so erhaltene gelbe Oel (ca. 4 g) wurde in 100 ml Acetonitril gelöst, mit 150 mg Triphenylphosphin versetzt und nach Erwärmen auf 50°C 10 ml einer 0,125%igen Palladium(II)-nitrat-Lösung in Acetonitril hinzugetropft. Nach 2-stündigem Erwärmen auf 50°C wurde das Reaktionsgemisch auf 0°C abgekühlt. Nach 2 Stunden wurden die ausgefallenen Kristalle abgesaugt und aus Hexan/Essigester umkristallisiert. Man erhielt 1,4 g (2Z,4E,6E,8E)-9-(5-Oxo-2,6,6-trimethyl-cyclohexa-1,3-dienyl)-3,7-dimethyl-nona-2,4,6,8-tetraensäure in gelben Kristallen, Smp. 188°C (Zers.).

Beispiel 5

7 g [(2E,4E)-3-Methyl-5-(2,6,6-trimethyl-5-oxo-1-cyclohexen-1-yl)-2,4-pentadienyl]triphenylphosphoniumbromid und 1,4 g 5-Hydroxy-4-methyl-5H-furan-2-on wurden in 100 ml Isopropanol suspendiert. Nach dem Abkühlen des Reaktionsgemisches auf -30°C wurde eine Lösung von 1,76 g Kaliumhydroxid in 30 ml Isopropanol hinzugetropft. Nach 40-minütigem Rühren bei -30°C wurde das Reaktionsgemisch auf 200 ml Eiswasser gegossen, 6 mal mit einem Hexan/Essigester-Gemisch (2:1) extrahiert, die organischen Phasen mit Wasser gewaschen, die vereinigten, wässrigen Phasen mit 3N Schwefelsäure angesäuert und mehrfach mit Essigester extrahiert. Die organische Phase wurde gewaschen ($H_2O$), getrocknet ($Na_2SO_4$) und eingedampft. Der ölige Rückstand wurde in 100 ml Essigester gelöst, mit 12 g Kieselgel versetzt und bei 40°C unter Argon kräftig gerührt. Nach dem Abkühlen wurde vom Kieselgel abfiltriert, gut mit Essigester nachgewaschen und eingedampft. Der Rückstand wurde in 30 ml Acetonitril gelöst und nach Zugabe von 256 mg Triphenylphosphin bei 50°C tropfenweise mit einer Lösung von 26 mg Palladiumnitrat in 5 ml Acetonitril versetzt. Nach 1-stündigem Rühren bei 50°C wurde das Reaktionsgemisch auf 0°C gekühlt und nach 1 Stunde der ausgefallene Niederschlag abfiltriert. Nach 2 maligem Umkristallisieren aus Hexan/Essigester erhielt man 1,4 g (2Z,4E,6E,8E)-3,7-Dimethyl-9-(2,6,6-trimethyl-5-oxo-cyclohex-1-enyl)-nona-2,4,6,8-tetraensäure in gelben Kristallen, Smp. 178-179°C.

Beispiel 6

0,5 g (2Z,4E,6E,8E)-3,7-Dimethyl-9-(2,6,6-trimethyl-5-oxo-cyclohex-1-enyl)-nona-2,4,6,8-tetraensäure wurden unter leichtem Erwärmen in 25 ml Ethanol gelöst und unter Eiskühlung mit 100 mg Natriumborhydrid versetzt. Nach 1-stündigem Rühren bei 0°C wurde das Reaktionsgemisch auf Eiswasser gegossen, mit 2N Salzsäure angesäuert und mit Essigester extrahiert. Die organischen Phasen wurden gewaschen ($H_2O$), getrocknet ($Na_2SO_4$) und eingedampft. Der amorphe Rückstand wurde über eine kleine Säule (Kieselgel, Hexan/Essigester = 1:1) filtriert und aus Hexan/Essigester umkristallisiert. Man erhielt 0,35 g (2Z,4E,6E,8E)-9-(5-Hydroxy-2,6,6-trimethyl-cyclohex-1-enyl)-3,7-dimethyl-nona-2,4,6,8-tetraensäure in gelben Kristallen, Smp. 152-154°C.

Beispiel 7

2,5 g des nach Beispiel 2 hergestellten Ethylesters wurden in 35 ml abs. Tetrahydrofuran gelöst und nacheinander bei 0°C mit 3,8 g Triphenylphosphin, 2,4 g p-Nitrobenzoesäure und tropfenweise mit einer Lösung von 2,5 g Azodicarbonsäure-diethylester in 30 ml Tetrahydrofuran versetzt. Nach 2-stündigem Rühren bei Raumtemperatur wurde eingedampft, der Rückstand mit Ether aufgenommen und 3 Stunden im Kühlschrank aufbewahrt. Der dabei ausgefallene Niederschlag (Triphenylphosphinoxid) wurde abgetrennt, das Filtrat eingeengt und durch Chromatographie (Kieselgel, Hexan/Essigester 5%) gereinigt. Man erhielt 0,68 g eines gelben Oeles, das in 15 ml Ethanol gelöst und nach Zugabe einer Lösung von 0,78 g Kaliumhydroxid in je 3 ml Wasser, Ethanol und Tetrahydrofuran bei 45°C während 6 Stunden gerührt wurde. Das Reaktionsgemisch wurde anschliessend auf Eiswasser gegossen, mit 1N Salzsäure angesäuert und mit Essigester extrahiert. Die organische Phase wurde gewaschen ($H_2O$), getrocknet ($Na_2SO_4$) und eingedampft. Der kristalline Rückstand wurde über eine Kleine Säule (Kieselgel, Essigester) filtriert und ergab nach Umkristallisieren aus Hexan/Essigester 190 mg (2Z,4E,6E,8E)-(S)-9-(4-Hydroxy-2,6,6-trimethyl-cyclohex-1-enyl)-3,7-dimethyl-nona-2,4,6,8-tetraensäure in gelben Kristallen, Smp. 178-180°C, $[\alpha]_D^{20}$ = +48,2 (c = 0,66, Dioxan).

Beispiel 8

1 g der nach Beispiel 4 hergestellten Carbonsäure wurde in 50 ml Methylethylketon suspendiert, mit 1,5 g feingemahlenem Kaliumcarbonat und 1,3 ml Ethyljodid versetzt und während 1 Stunde am Rückfluss erwärmt. Das abgekühlte Reaktionsgemisch wurde auf Eiswasser gegossen, mit 1N Salzsäure neutralisiert, mit Essigester extrahiert, gewaschen ($H_2O$), getrocknet ($Na_2SO_4$) und eingedampft. Das so erhaltene gelbe Oel wurde durch Chromatographie (Kieselgel, Hexan/Essigester = 2:1) gereinigt und ergab 1,5 g des entsprechenden Ethylesters als gelbes Oel.

1,18 g Certrichlorid • $7H_2O$ wurden in 25 ml Methanol gelöst und nacheinander mit einer Lösung des obigen Ethylesters in 5 ml Methanol und 120 mg Natriumborhydrid versetzt. Nach dem Abklingen der Gasentwicklung (ca. 5 Minuten) wurde eingedampft und der orange Rückstand durch Chromatographie (Kieselgel, Hexan/Essigester =4:1) gereinigt. Man erhielt 1,04 g des entsprechenden Hydroxy-esters als gelbes Oel. Dieses Oel wurde in 25 ml Ethanol gelöst, mit einer Lösung von 1,6 g Kaliumhydroxid in 12 ml Wasser versetzt und während 4 Stunden bei 40°C unter Argon gerührt. Das abgekühlte Reaktionsgemisch wurde auf Eiswasser gegossen, mit 1N Salzsäure auf pH 3 angesäuert und mit Essigester extrahiert. Die organische Phase wurde gewaschen ($H_2O$), getrocknet ($Na_2SO_4$) und eingedampft und ergab 0,95 g eines orangen, amorphen Rückstandes. Filtration über eine Kleine Säule (Kieselgel, Hexan/Essigester = 1:1) und Kristallisation aus Hexan/Essigester ergab (2Z,4E,6E,8E)-9-(5-Hydroxy-2,6,6-trimethyl-cyclohexa-1,3-dienyl)-3,7-dimethyl-nona-2,4,6,8-tetraensäure in gelborangen Kristallen, Smp. 108-110°C.

Beispiel A

Hartgelatinekapseln können wie folgt hergestellt werden:

| Bestandteile | mg/Kapsel |
|---|---|
| 1. sprühgetrocknetes Pulver enthaltend 75% Verbindung I | 20 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 120 |

Das sprühgetrocknete Pulver, das auf dem Wirkstoff, Gelatine und mikrokristalliner Cellulose basiert und eine mittlere Korngrösse des Wirkstoffes von <1 μm aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

Beispiel B

Tabletten können wie folgt hergestellt werden:

| Bestandteile | mg/Tablette |
|---|---|
| 1. Verbindung I als feingemahlenes Pulver | 20 |
| 2. Milchzucker pulv. | 100 |
| 3. Maisstärke weiss | 60 |
| 4. Povidone K30 | 8 |
| 5. Maisstärke weiss | 112 |
| 6. Talk | 16 |
| 7. Magnesiumstearat | 4 |
| | Total 320 |

Die feingemahlene Substanz wird mit Milchzucker und einem Teil der Maisstärke gemischt. Die Mischung wird

mit einer wässrigen Lösung von Povidone K30 befeuchtet und geknetet, und die resultierende Masse granuliert, getrocknet und gesiebt. Das Granulat wird mit der restlichen Maisstärke, Talk und Magnesiumstearat vermischt und zu Tabletten geeigneter Grösse verpresst.

Beispiel C

Eine Lotion kann wie folgt hergestellt werden:

| Bestandteile | |
|---|---|
| 1. Verbindung I, feingemahlen | 1,0 g |
| 2. Carbopol 934 | 0,6 g |
| 3. Natriumhydroxid | q.s. ad pH 6 |
| 4. Aethanol, 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol 934 wird bis zur vollständigen Gelierung eingerührt und der pH-Wert mit Natriumhydroxid eingestellt.

Beispiel D

Eine Crème kann aus den nachstehend aufgeführten Inhaltsstoffen in an sich bekannter Weise hergestellt werden:

| | Gew.-% |
|---|---|
| Verbindung der Formel I | 0,1-5 |
| Cetylalkohol | 5,25-8,75 |
| Arlacel 165 (Glyceryl/PEG 100-stearat) | 3,75-6,25 |
| Miglyol 818 (Capryl-/Caprin-/Linolsäure triglycerid) | 11,25-18,75 |
| Sorbit-Lösung | 3,75-6,25 |
| EDTA-$Na_2$ | 0,075-0,125 |
| Carbopol 934P (Carbomer 934P) | 0,15-0,25 |
| butyliertes Hydroxyanisol | 0,0375-0,0625 |
| Methylparaben | 0,135-0,225 |
| Propylparaben | 0,0375-0,0625 |
| NaOH (10% solution) | 0,15-0,25 |
| Wasser q.s. | 100,00 |

Beispiel E

Ein Gel kann aus den nachstehend aufgeführten Inhaltsstoffen in an sich bekannter Weise hergestellt werden:

| | Gew.-% |
|---|---|
| Verbindung der Formel I | 0,1-5 |
| Pluronic L 101 (Poloxamer 331) | 10,00 |
| Aerosil 200 (Siliciumdioxid) | 8,00 |
| PCL Liquid (Fettsäureester | 15,00 |
| Cetiol V (Decyloleat) | 20,00 |
| Neobee Oil (Triglycerid mittlerer Kettenlänge) | 15,00 |
| Euhanol G (Octyldodecanol), q.s. | 100,00 |

Durch Variation der Verhältnisse zwischen den Hilfsstoffen der Beispiele D und E können die physikalischen Eigenschaften der Präparate verändert werden.

## Patentansprüche

1. Verbindungen der allgemeinen Formel

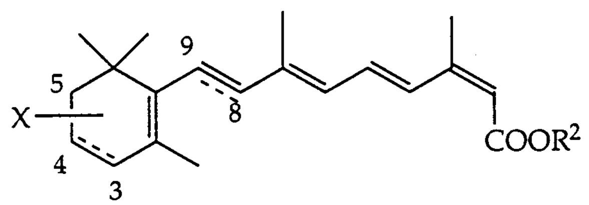

I

worin

X Oxo oder ein Rest $OR^1$ in 4- oder 5-Stellung;
$R^1$ Wasserstoff, Alkyl oder Acyl; und
$R^2$ Wasserstoff oder Alkyl ist;

die punktierten Bindungen fakultativ sind und eine 3,4-Doppelbindung nur anwesend sein kann, wenn X in 5-Stellung ist;
sowie pharmazeutisch anwendbare Salze von Carbonsäuren der Formel I.

2. Verbindungen gemäss Anspruch 1 der Formel

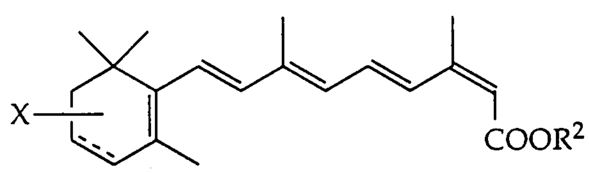

IA

3. Verbindungen gemäss Anspruch 1 der Formel

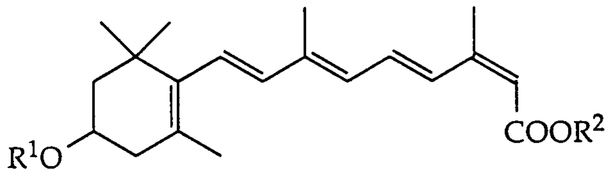

IB

4. Verbindungen gemäss Anspruch 1 der Formel

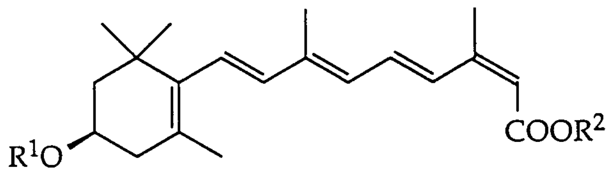

IC

5. (2Z,4E,6E,8E)-(R-)-9-(4-Hydroxy-2,6,6-trimethyl-cyclohex-1-enyl)-3,7-dimethyl-nona-2,4,6,8-tetraensäure.

6. (2Z,4E,6E,8E)-(R)-9-(4-Hydroxy-2,6,6-trimethyl-cyclohex-1-enyl)-3,7-dimethyl-nona-2,4,6,8-tetraensäure-ethylester.

7. (2Z,4E,6E,8E)-(S)-9-(4-Hydroxy-2,6,6-trimethyl-cyclohex-1-enyl)-3,7-dimethyl-nona-2,4,6,8-tetraensäure.

8. Verbindungen gemäss Anspruch 1 der Formel

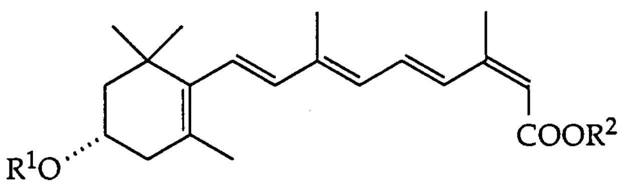

ID

9. Verbindungen gemäss Anspruch 1 der Formel

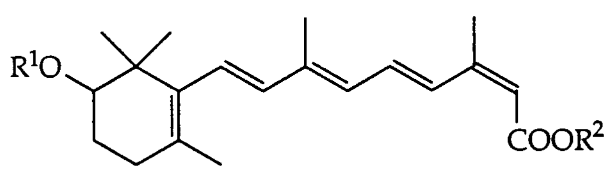

IE

10. (2Z,4E,6E,8E)-9-(5-Hydroxy-2,6,6-trimethyl-cyclohex-1-enyl)-3,7-dimethyl-nona-2,4,6,8-tetraensäure.

11. Verbindungen gemäss Anspruch 1 der Formel

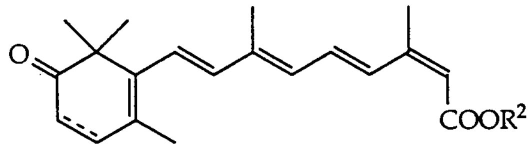

IF

12. (2Z,4E,6E,8E)-9-(5-Oxo-2,6,6-trimethyl-cyclohexa-1,3-dienyl)-3,7-dimethyl-nona-2,4,6,8-tetraensäure.

13. (2Z,4E,6E,8E)-3,7-Dimethyl-9-(2,6,6-trimethyl-5-oxo-cyclohex-1-enyl)-nona-2,4,6,8-tetraensäure.

14. Verbindungen gemäss Anspruch 1 der Formel

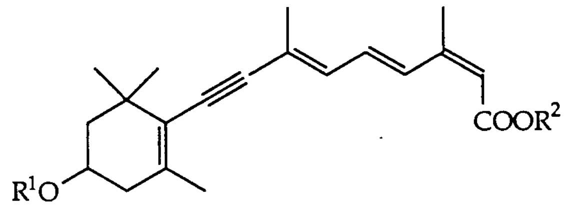

IG

15. (2Z,4E,6E)-(R)-9-(4-Hydroxy-2,6,6-trimethyl-cyclohex-1-enyl)-3,7-dimethyl-nona-2,4,6-trien-8-yn-säure.

16. Die Verbindungen der Ansprüche 1-15 zur Anwendung als Heilmittel, besonders zur Anwendung bei Akne, Psoriasis, licht- und altersgeschädigter Haut, sowie malignen und prämalignen Epithelläsionen.

17. Pharmazeutische Präparate, enthaltend eine Verbindung der Ansprüche 1-15 und übliche pharmazeutische Trägerstoffe.

18. Verwendung von Verbindungen der Ansprüche 1-15 bei der Herstellung von Heilmitteln zur Therapie und Prophylaxe von Akne, Psoriasis, licht- und altersgeschädigter Haut, sowie malignen und prämalignen Epithelläsionen.

19. Verfahren zur Herstellung der Verbindungen der allgemeinen Formel I und pharmazeutisch anwendbaren Salzen davon, dadurch gekennzeichnet, dass man eine Verbindung der allgemeinen Formel

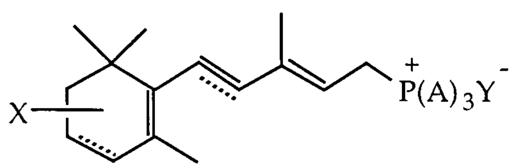

*II*

worin A Phenyl oder substituiertes Phenyl und $Y^-$ ein Anion ist,

und X und die punktierten Bindungen die oben angegebene Bedeutung haben,

mit 5-Hydroxy-4-methyl-5H-furan-2-on in Gegenwart einer Base umsetzt, im Reaktionsprodukt enthaltene Isomere, in denen die bei der Reaktion gebildete C=C-Doppelbindung cis-Konfiguration aufweist, zum trans-Isomeren isomerisiert und gewünschtenfalls das Reaktionsprodukt der Formel I einer oder mehreren der nachfolgenden Operationen unterwirft:

a) Veresterung einer Carboxyl- oder Hydroxygruppe;
b) Verätherung einer Hydroxygruppe;

c) Oxidation einer Hydroxygruppe zur Oxogrupppe;
d) Reduktion einer Oxogruppe zur Hydroxygruppe;
e) sterische Invertierung einer Hydroxygruppe; und
f) Ueberführung einer Carboxygruppe in ein Salz.

## Claims

1. Compounds of the general formula

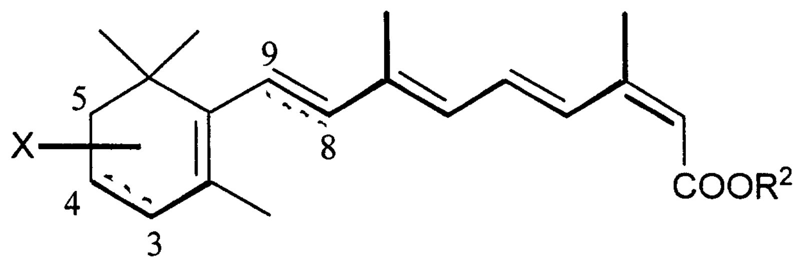

I

wherein

X is oxo or a residue $OR^1$ in the 4- or 5-position;
$R^1$ is hydrogen, alkyl or acyl; and
$R^2$ is hydrogen or alkyl;

the dotted bonds are optional and a 3,4-double bond can be present only when X is in the 5-position; as well as pharmaceutically usable salts of carboxylic acids of formula I.

2. Compounds according to claim 1 of the formula

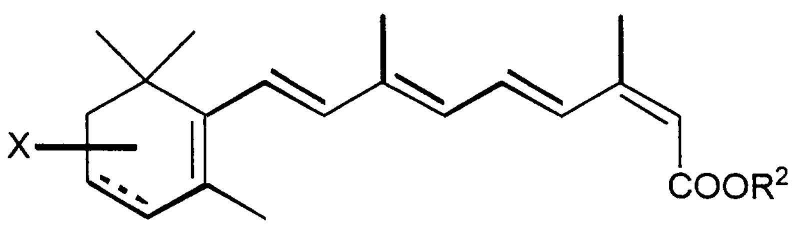

IA

3. Compounds according to claim 1 of the formula

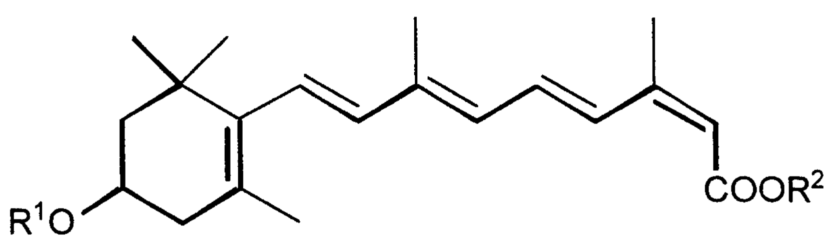

IB

4. Compounds according to claim 1 of the formula

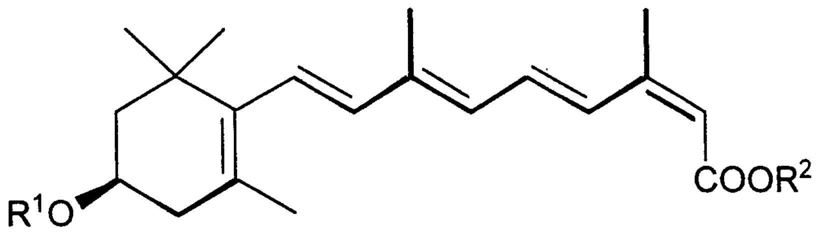

IC

5. (2Z,4E,6E,8E)-(R)-9-(4-Hydroxy-2,6,6-trimethyl-cyclohex-1-enyl)-3,7-dimethyl-nona-2,4,6,8-tetraenoic acid.

6. Ethyl (2Z,4E,6E,8E)-(R)-9-(4-hydroxy-2,6,6-trimethyl-cyclohex-1-enyl)-3,7-dimethyl-nona-2,4,6,8-tetraenoate.

7. (2Z,4E,6E,8E)-(S)-9-(4-Hydroxy-2,6,6-trimethyl-cyclohex-1-enyl)-3,7-dimethyl-nona-2,4,6,8-tetraenoic acid.

8. Compounds according to claim 1 of the formula

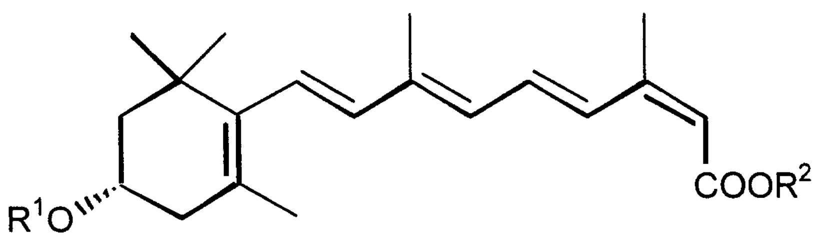

ID

9. Compounds according to claim 1 of the formula

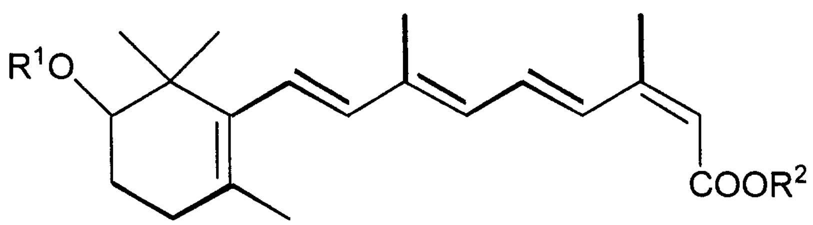

IE

10. (2Z,4E,6E,8E)-9-(5-Hydroxy-2,6,6-trimethyl-cyclohex-1-enyl)-3,7-dimethyl-nona-2,4,6,8-tetraenoic acid.

11. Compounds according to claim 1 of the formula

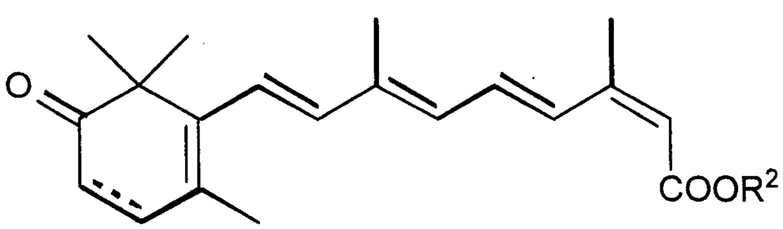

IF

**12.** (2Z,4E,6E,8E)-9-(5-Oxo-2,6,6-trimethyl-cyclohexa-1,3-dienyl)-3,7-dimethyl-nona-2,4,6,8-tetraenoic acid.

**13.** (2Z,4E,6E,8E)-3,7-Dimethyl-9-(2,6,6-trimethyl-5-oxo-cyclohex-1-enyl)-nona-2,4,6,8-tetraenoic acid.

**14.** Compounds according to claim 1 of the formula

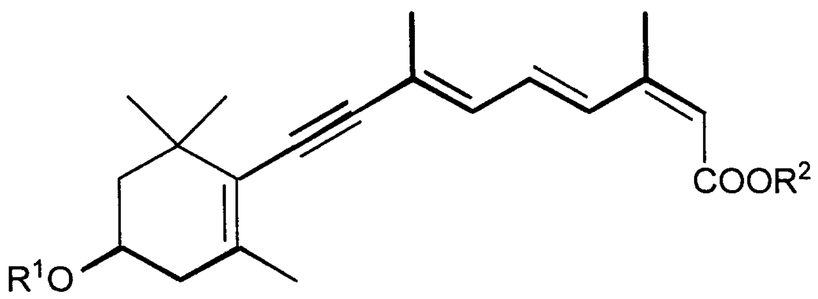

IG

**15.** (2Z,4E,6E)-(R)-9-(4-Hydroxy-2,6,6-trimethyl-cyclohex-1-enyl)-3,7-dimethyl-nona-2,4,6-trien-8-ynoic acid.

**16.** The compounds of claims 1-15 for use as medicaments, especially for use in acne, psoriasis, light- and age-damaged skin as well as malignant and pre-malignant epithelial lesions.

**17.** Pharmaceutical preparations containing a compound of claims 1-15 and conventional pharmaceutical carrier materials.

**18.** Use of compounds of claims 1-15 for the production of medicaments for the therapy and prophylaxis of acne, psoriasis, light- and age-damaged skin as well as malignant and pre-malignant epithelial lesions.

**19.** A process for the manufacture of the compounds of general formula I and pharmaceutically usable salts thereof, characterized by reacting a compound of the general formula

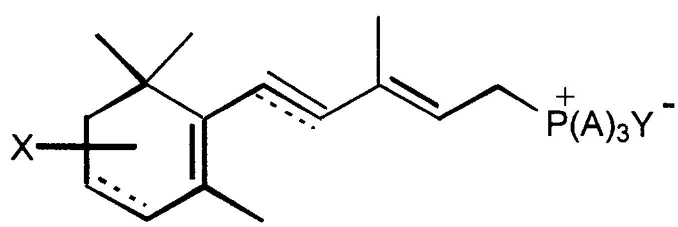

II

wherein A is phenyl or substituted phenyl and $Y^-$ is an anion, and X and the dotted bonds have the significance given above,
with 5-hydroxy-4-methyl-5H-furan-2-one in the presence of a base, isomerizing isomers present in the reaction product in which the C=C double bond formed in the reaction has the cis configuration to the trans isomers and, if desired, subjecting the reaction product of formula I to one or more of the following operations:

a) esterification of a carboxyl or hydroxy group;
b) etherification of a hydroxy group;
c) oxidation of a hydroxy group to the oxo group;
d) reduction of an oxo group to the hydroxy group;
e) steric inversion of a hydroxy group; and
f) conversion of a carbon group into a salt.

## Revendications

1. Composé de formule générale

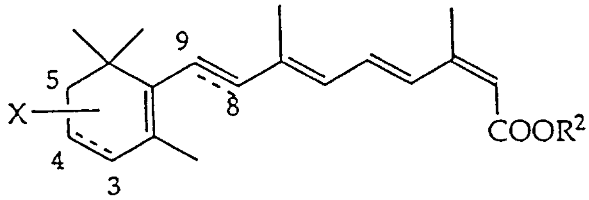

I

dans laquelle

X est un oxo ou un radical $OR^1$ en position 4 ou 5 ;
$R^1$ représente un hydrogène, un alkyle ou un acyle ; et
$R^2$ représente un hydrogène ou un alkyle ;

les liaisons en pointillé sont facultatives et une double liaison en 3,4 ne peut être présente que lorsque X est en position 5 ;
ainsi que les sels pharmaceutiquement acceptables des acides carboxyliques de formule I.

2. Composés selon la revendication 1 de formule

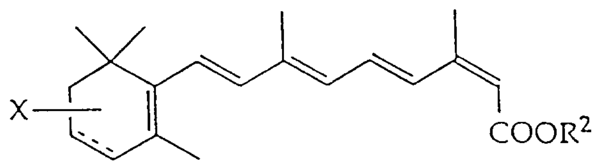

IA

3. Composés selon la revendication 1 de formule

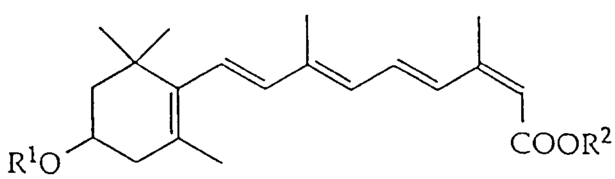

IB

4. Composés selon la revendication 1 de formule

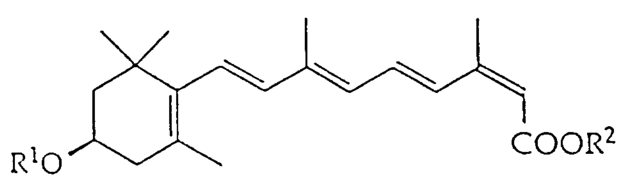

IC

5. Acide (2Z,4E,6E,8E)-(R)-9-(4-hydroxy-2,6,6-triméthyl-cyclohex-1-ényl)-3,7-diméthyl-nona-2,4,6,8-tétraénoïque.

6. Ester éthylique de l'acide (2Z,4E,6E,8E)-(R)-9-(4-hydroxy-2,6,6-triméthyl-cyclohex-1-ényl)-3,7-diméthyl-nona-2,4,6,8-tétraénoïque.

7. Acide (2Z,4E,6E,8E)-(S)-9-(4-hydroxy-2,6,6-triméthyl-cyclohex-1-ényl)-3,7-diméthyl-nona-2,4,6,8-tétraénoïque.

8. Composés selon la revendication 1 de formule

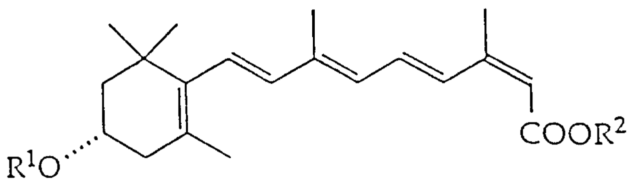

ID

9. Composés selon la revendication 1 de formule

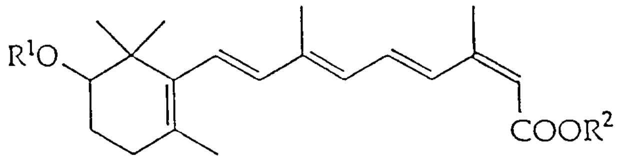

IE

10. Acide (2Z,4E,6E,8E)-9-(5-hydroxy-2,6,6-triméthyl-cyclohex-1-ényl)-3,7-diméthyl-nona-2,4,6,8-tétraénoïque.

11. Composés selon la revendication 1 de formule

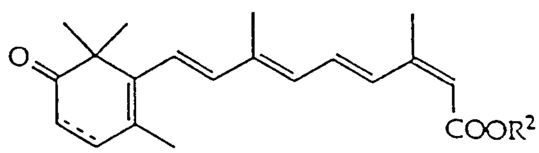

IF

12. Acide (2Z,4E,6E,8E)-9-(5-oxo-2,6,6-triméthyl-cyclohexa-1,3-diényl)-3,7-diméthyl-nona-2,4,6,8-tétraénoïque.

13. Acide (2Z,4E,6E,8E)-3,7-diméthyl-9-(2,6,6-triméthyl-5-oxo-cyclohex-1-ényl)-nona-2,4,6,8-tétraénoïque.

14. Composés selon la revendication 1 de formule

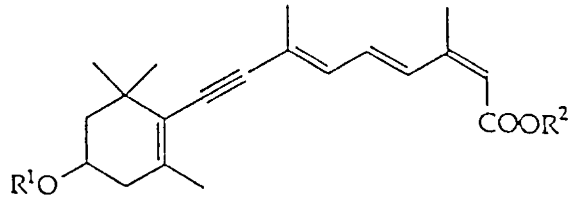

IG

15. Acide (2Z,4E,6E)-(R)-9-(4-hydroxy-2,6,6-triméthyl-cyclohex-1-ényl)-3,7-diméthyl-nona-2,4,6-trién-8-ynoïque.

16. Composés selon les revendications 1-15 aux fins d'application code médicaments, en particulier aux fins d'application dans l'acné, le psoriasis, sur la peau endommagée par la lumière et l'âge, ainsi que dans les lésions épithéliales malignes et prémalignes.

17. Préparations pharmaceutiques contenant un composé selon les revendications 1-15 et des supports pharmaceutiquement habituels.

18. Utilisation de composés selon les revendications 1-15 dans la préparation de médicaments pour le traitement et la prophylaxie de l'acné, du psoriasis, de la peau endommagée par la lumière et l'âge, ainsi que les lésions épithéliales malignes et prémalignes.

19. Procédé de préparation des composés de formule générale I et de leurs sels pharmaceutiquement acceptables, caractérisé en ce qu'on fait réagir un composé de formule générale

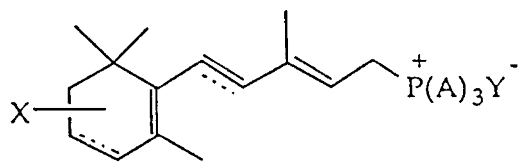

II

dans laquelle A représente un phényle ou un phényle substitué et $Y^-$ représente un anion, et X et les liaisons en pointillé ont la signification indiquée ci-dessus,
avec la 5-hydroxy-4-méthyl-5H-furan-2-one en présence d'une base, en ce qu'on isomérise les isomères contenus dans le produit de la réaction, dans lesquels la double liaison C=C formée lors de la réaction présente une configuration cis, pour obtenir l'isomère trans, et le cas échéant en ce qu'on soumet le produit réactionnel de formule I à une ou plusieurs des opérations suivantes :

a) estérification d'un groupe carboxyle ou hydroxy ;
b) éthérification d'un groupe hydroxy ;
c) oxydation d'un groupe hydroxy en groupe oxo ;
d) réduction d'un groupe oxo en groupe hydroxy ;
e) inversion stérique d'un groupe hydroxy ; et
f) transformation d'un groupe carboxy en un sel.